# EUROPEAN PATENT SPECIFICATION

(11) **EP 1 753 479 B1**
(45) Date of publication and mention of the grant of the patent: **05.08.2009**
(21) Application number: 05782619.0
(22) Date of filing: 29.04.2005
(51) Int. Cl.: A61L 29/04, A61L 29/12

(54) **EXPANDABLE MEDICAL BALLOONS AND METHODS OF PREPARING THE SAME**
EXPANDIERBARE MEDIZINISCHE BALLONS UND VERFAHREN ZU IHRER HERSTELLUNG
BALLONETS MEDICAUX EXPANSIBLES ET LEURS METHODES DE PREPARATION

(30) Priority: 07.06.2004 US 862250
(43) Date of publication of application: 21.02.2007
(73) Proprietor: Boston Scientific Limited, Christ Church (BB)
(72) Inventor: MAPES, Ken, Temecula, California 92592 (US); ERAMO, Lincoln, Winchester, California 92596 (US)
(74) Representative: Altenburg, Udo
(86) International application number: PCT/US2005/014827
(87) International publication number: WO 2005/120622

(56) References cited:
- US-A- 2 472 485
- US-A- 5 769 817
- US-A- 5 876 386
- US-B1- 6 206 854

## Description

### BACKGROUND OF THE INVENTION

Atherosclerotic cardiovascular disease is common, and is caused by a narrowing of the arterial lumen due to atherosclerotic plaques. Balloons mounted on the distal ends of catheters are commonly used in the medical treatment of atherosclerotic diseases. Such balloons may be used for dilating lesions or blockages by compressing plaque, for recanalizing and dilating a diseased vessel, and for expanding prosthetic devices such as stents at a desired location in a bodily vessel. The requirements for strength and size of the balloons vary widely depending on the balloon's intended use and the vessel size into which the catheter is inserted.

Percutaneous transluminal coronary angioplasty, or balloon angioplasty, is a non-invasive, non-surgical means of treating peripheral and coronary arteries. This technique consists of inserting an uninflated balloon catheter into the affected artery. Dilation of the diseased segment of artery is accomplished b the arterial diameter. Typically, inflation is repeated two additional times. The balloon is then deflated and the catheter is withdrawn.

Cutting balloons are another type of medical balloon which have cutting edges, also referred to as atherotomes or blades for recanalizing and dilating a diseased vessel, and facilitating balloon angioplasty procedures.

In either type of application, it is typically necessary for the balloon to traverse a tortuous anatomy as it is being delivered to the location in extremely small bodily vessels and used to open stenoses of blood vessels by balloon inflation. In these applications, it is desirable for the balloon to assume as low a profile, i.e. the outer diameter of the distal end portion of the balloon, as possible. Considerable effort has been put forth in the development of dilatation balloons with a low profile by minimizing the dimensions of the core or the inner tube which extends through the balloon to its distal end, and by reducing the wall thickness of the balloon itself.

Thus for such applications, thin walled, high strength, relatively inelastic balloons of predictable inflation properties are desired. However, this combination of properties, i.e. thin walls and low resilience, may have increased susceptibility to pin hole formation and ruptures.

US-A-5,796,817 discloses an expander member for a balloon catheter to be used in stent delivery applications, comprising a double-walled tubular member that is adapted for attachment to a distal end portion of an elongated, flexible, tubular catheter body.

There remains a need for a balloon having improved abrasion resistance and resistance to rupture during use, without sacrificing flexibility and while maintaining a thin-walled structure.

### SUMMARY OF THE INVENTION

The present specification describes the formation of expandable medical balloons, using a fiber web, and to the articles formed thereby.

While the present invention finds utility for balloons used for coronary angioplasty procedures, the present invention also finds utility for other types of medical balloons including, but not limited to, cutting balloons, balloons used in the biliary duct, urinary tract, expandable members for medical delivery devices including stents, etc.

In one aspect, the present specification describes application of the fiber web provided over a first layer. The first layer may be formed from any suitable polymeric composition, as well as ceramic, metal, or the like. Suitable polymeric materials include thermoplastic and thermosetting polymeric materials, as well as elastomers and non-elastomers. The first layer may define the shape of the medical device.

Optionally, a composition for increasing the friction between the first layer and the web, or other wise adhesively binds the first layer and the web, may be employed herein. Such a composition may be hereinafter referred to as an adhesive composition.

Additionally, a matrix coating for encapsulation or embedding of the fiber may be employed on the inner and/or outer surface of the fiber web. This may be applied alternatively to, or in addition to the adhesive composition. The matrix material may comprise any suitable material which may be applied for a variety of reasons such as preventing penetration of water/saline, to fill in areas around the fiber of the web, to encapsulate the web, to increase balloon integrity and improve abrasion/puncture resistance, and to increase lubricity, for example. This coating may also be designed such that lubricity is provided to the surface, and may also carry therapeutic agent(s) such as an anticoagulant. Thermoplastic and thermosetting materials, and fibrous materials are suitable, as well as mixtures thereof. The coating may be applied using any suitable means known in the art including, but not limited to, spraying, dipping, brushing and so forth.

In an embodiment in which the fiber web defines the shape of the medical device, it may be desirable to apply a matrix material to both the inner and outer surface of the fiber web. Of course, the coating on the inner and outer surface need not be the same.

A shape-form which is eliminated after use by fluidization, or by other techniques, may be employed in making the balloons according to the invention.

The present invention allows for tailoring of physical properties to the demands of the article being formed. For example, the resultant medical devices can be designed for flexibility, strength, lubricity and for having resistance to abrasions and tearing, i.e. "rip-stop" characteristics.

In particular, the present invention provides an expandable medical balloon, the expandable medical balloon comprising:
a) a first layer having an inner surface and an outer surface defining the shape of the expandable balloon member; and
b) a web formed with silk fiber having an inner surface and an outer surface, the web provided over at least a portion of said inner surface, said outer surface or both of said first layer.

Other aspects of the invention are described in the Detailed Description and in the claims below.

### BRIEF DESCRIPTION OF THE DRAWINGS

FIG. 1 is longitudinal cross-sectional side view of a catheter assembly having a balloon of the present invention mounted thereon.
FIG. 2 is a partial cross-sectional view taken at part B in FIG. 1.
FIG. 3 is a cross-sectional view of a balloon according to the invention.
FIG. 4 is a cross-sectional view taken at part A in FIG. 3.
FIG. 5 is a side view of a balloon.
FIG. 6 is a side view of a pre-formed fiber web.
FIG. 7 is a perspective view of a balloon structure as in FIG. 3 shown being inserted into a pre-formed fiber web as in FIG. 5.
FIG. 8 is a side view of a balloon in a deflated state.
FIG. 9 is a side view of a chopped fiber mat.

FIG. 10 is a detailed partial cross-sectional view of the fiber mat shown in FIG. 9.
FIG. 11 is a perspective side view of an inflated balloon shown inserted in a chopped fiber mat.
FIG. 12 is an alternate perspective view of the balloon and fiber mat of FIG. 11.
FIG. 13 is a perspective side view of an inflated balloon inserted in a fiber web.
FIG. 14 is an alternate perspective view of the balloon and fiber web as shown in FIG. 13.
FIG. 15 is a detailed partial cross-sectional view of the balloon and fiber web of FIGS. 13 and 14.
FIG. 16 is a perspective side view of a cutting balloon of the invention. FIG. 17 is a detailed partial cross-sectional view taken from the balloon as shown in FIG. 16.

### DETAILED DESCRIPTIONS OF THE INVENTION

The present specification describes the use of a fiber web in the formation of expandable medical balloons,
wherein a fiber web is provided over at least one first layer of the medical device, the first layer defining the shape of the medical device. The fiber web may be applied over the inner surface of the first layer, the outer surface of the first layer, or both.

In one embodiment, the first layer is eliminated after use, leaving the fiber web to define the shape of the medical device.

Other embodiments of the invention are described in more detail below.

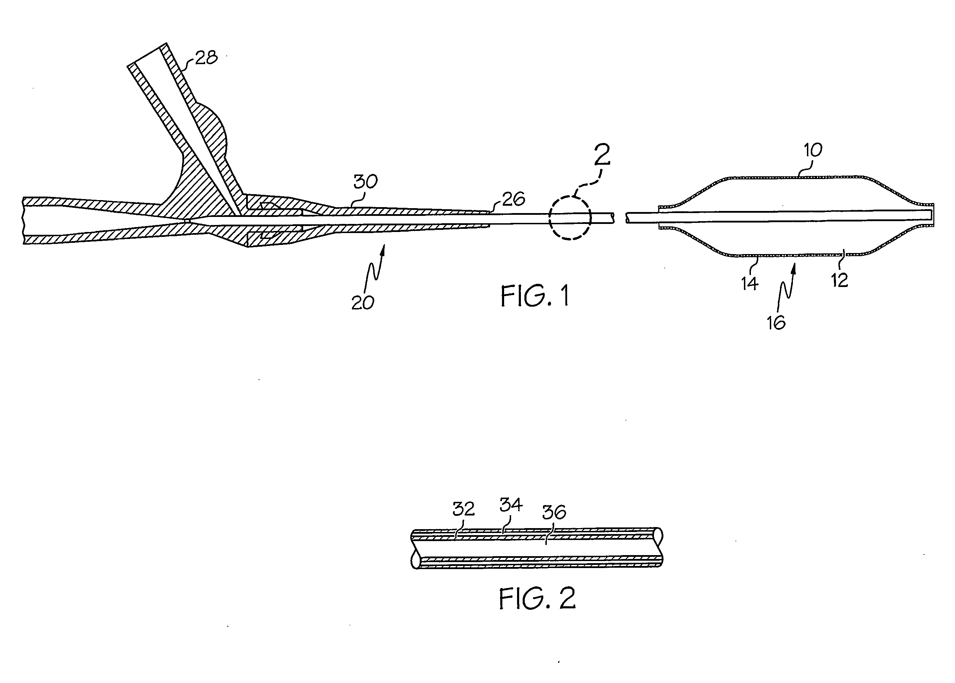

Referring now to the figures, FIG. 1 shows balloon 10 in accordance with one aspect of the invention, in combination with a catheter assembly 20. In this embodiment, balloon 10 is shown having a first layer 12, a web of fiber 14 and matrix coating 16 over said web of fiber 14. Catheter 20 is a representative over-the-wire (OTW) or single-operator-exchange (SOE) angioplasty balloon catheter according to the invention. Such balloon catheters are discussed, for example, in commonly assigned US Patent No. 6113579, 6517515, 6514228.

In this embodiment, catheter 20 has an elongate shaft assembly 26 and a conventional OTW-type manifold assembly 28 connected to proximal end of shaft assembly 26. Manifold assembly 28, is further shown with a strain relief 30. The shaft assembly 26 includes an inner tube 32 and an outer tube 34. Outer tube 34 is coaxially disposed about inner tube 32 to define an annular inflation lumen 36 shown in cross-section in FIG. 2. This is only an illustration of such a catheter assembly and is not intended to limit the scope of the present invention. Numerous structures are known to those of skill in the art, any of which may be employed herein.

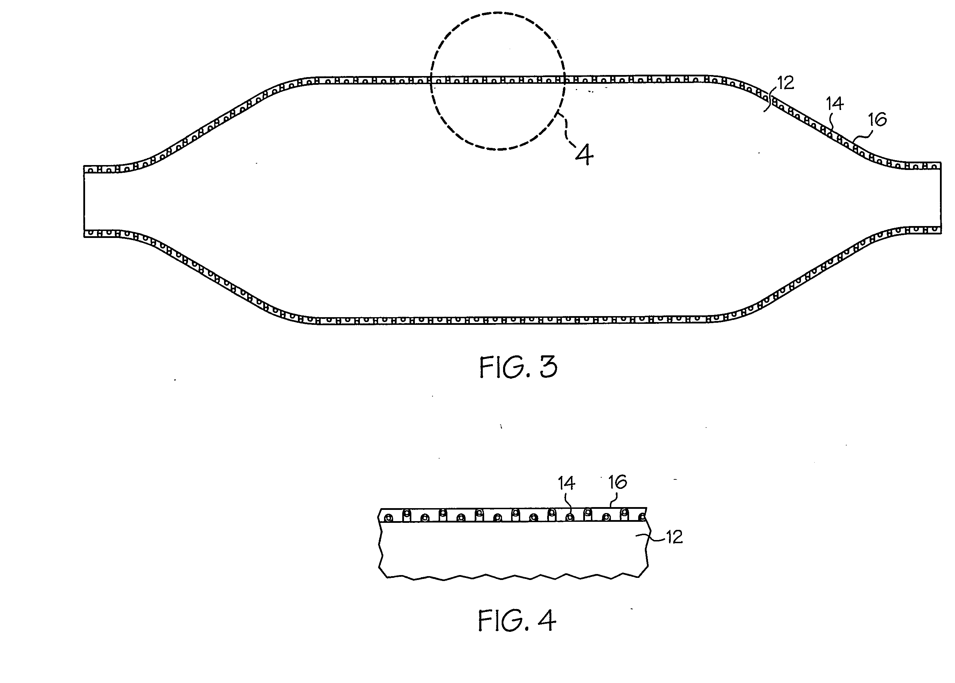

Balloon 10 according to the invention may be constructed in a variety of ways. FIG. 3 is a cross-sectional side-view of one embodiment of balloon 10. Balloon 10 is shown with a first layer 12 which defines the shape of balloon 10, a fiber 14 and a matrix coating 16.

Balloon 10 may also be formed using a shape-form (not shown in FIG. 3) which defines the shape of balloon 10, but which is eliminated after use. The first layer 12 may be provided over the eliminatable shape form followed by fiber web 14 and matrix 16, or, fiber 14 may be provided over an eliminatable shape form followed by matrix coating 16, without having a first layer 12. The inner surface of the fiber web may also be coated with the same matrix coating, or may be coated with a different coating composition. Such embodiments are discussed in more detail below.

A first layer 12 defining the shape of balloon 10 may be formed from any suitable polymeric material known in the art including thermoplastic materials and thermosetting materials such as moisture curable and radiation curable monomers, oligomers and polymers, as well as elastomers and non-elastomers.

Examples of non-elastomeric materials include, but are not limited to, polyolefins, polyesters, polyethers, polyamides, polyurethanes, polyimides, copolymers and terpolymers thereof, and so forth. As used herein, the term "copolymer" shall be hereinafter be used to refer to any polymer formed from two or more monomers.

Examples of suitable elastomeric materials include, but are not limited to, elastomeric block copolymers including the block copolymers having styrene endblocks and diene midblocks such as such as styrene-ethylene/butylene-styrene (SEBS) block copolymers disclosed in US Patent 5112900.

Other suitable block copolymer elastomers include, but are not limited to, styrene-isoprene-styrene (SIS), styrene-butadiene-styrene (SBS), and so forth.

Block copolymer elastomers are also described in commonly assigned US Patent Nos. 6406457, 6171278, 6146356, 5951941, 5830182, 5556383.

Elastomeric polyesters and copolyesters may be employed herein. Examples of elastomeric copolyesters include, but are not limited to, poly(ester-block ether) elastomers, poly(ester-block-ester) elastomers and so forth. Poly(ester-block-ether) elastomers are available under the tradename of HYTREL® from DuPont de Nemours & Co. and consist of hard segments of polybutylene terephthalate and soft segments based on long chain polyether glycols. These polymers are also available from DSM Engineering Plastics under the tradename of ARNITEL®.

Non-elastomeric polyesters and copolymers thereof may be employed such as the polyalkylene naphthalates may be employed herein including polyethylene terephthalates and polybutylene terephthalates, for example.

Polyamides including nylon, and copolymers thereof such as poly (ether-block-amides) such as those available under the tradename of PEBAX® available from Atofina Chemicals in Philadelphia, PA.

Suitable balloon materials are described in commonly assigned U.S. Patent Nos. 5549552, 5447497, 5348538, 5550180, 5403340, 6328925.

The above lists are intended for illustrative purposes only, and not as a limitation on the scope of the present invention. Other polymeric materials not described herein, may find utility in the formation of catheter balloons according to the invention.

The balloons according to the present invention may be formed using any conventional methods known in the art. Any suitable balloon forming techniques may be employed. Such techniques are known in the art. An example of one method is described in US 4,490,421 to Levy.

The methods typically include the basic steps of extruding a tubular parison or balloon preform, placing the tubular parison in a balloon mold, and expanding the tubular parison into the desired balloon configuration in the balloon mold. The main processing steps may include other steps therein such as stretching and radial orientation of the balloon material, for example, as well as annealing and heat setting.

The resultant balloons may have a single wall thickness, prior to addition of the fiber web, or further coatings, of about 10 to about 50 microns, but this may vary depending on the application for which the balloon is employed. For example, a typical angioplasty balloon may have a *total* wall thickness of about 20 to about 30 microns, including each of the additional layers. However, such limits may vary outside of these parameters, as they are only given for illustrative purposes.

One illustration of a balloon according to the invention, may be one which
wherein the first layer is 12 microns, a first web of the fiber is 12 microns and a second web of fiber is 12 microns for a total wall thickness of 36 microns.

As shown in FIG. 3, balloon 10 further has a fiber 14 applied over the first layer 12 forming a web structure over the outer surface of balloon 10.

As used herein, the term "fiber" may be used interchangeably with thread, yam and so forth. Each fiber may be made of a monofilament, or each fiber may include multiple filaments, i.e. a multi-filament fiber.

Suitable fibers for use herein include silk fibers and include both synthetic and natural fibers. As used herein, natural fibers refer to those which occur in nature, i.e. those produced by members of the phylum Arthropoda including arachnids and insects such as spiders, silk worms, black flies, wasps, and lacewing flies, while synthetic fibers refer to those fibers which are man-made such as those produced using recombinant protein technology. Hereinafter, the term "insects" shall be used to refer to those Furthermore, monofilament silk as well as multi-filament fiber may be employed.

Synthetic spider silk may be produced using recombinant protein technology. Recombinant spider silk protein has been found to produce fiber having a suitable balance of properties including flexibility, strength and biocompatibility. One example of a suitable fiber is that available from Nexia Biotechnology located at 1000 St-Charles Avenue, Block B, Vaudreuil-Dorion, QC, J7V 8P5 Canada under the tradename of BioSteel®, a protein-based biopolymer filament that is man-made. This is a recombinant spider silk protein produced in the milk if transgenic goats which is then purified and spun into fibers. BioSteel® silk is strong, waterproof and stretchable as well as having suitable biocompatibility.

Furthermore, Nexia produces BioSteel® spider silk fiber using a dragline spider silk structure. Dragline silk is that which comprises the radiating spokes of a spider web, and is from which the spiders make the scaffolding of their webs. Dragline silk has an excellent combination of strength and flexibility.

Other sources of recombinant spider silk protein include bacteria, for example *E*. *coli*, and yeast.

One source of natural spider silk is from a spider species called *Nephila edulis* with the addition of potassium chloride. See *NMR Chacterization of Native Liquid Spider Dragline Silk from Nephila edulis* [Hronska et al.; "NMR Chacterization of Native Liquid Spider Dragline Silk from Nephila edulis "; Biomacromolecules 2002, 3, 644-648 (http://www.nmr.ethz.ch/∼piwi/publications.html)].

Another source of natural spider silk is from the silkworm and is referred to as regenerated silkworm silk. Silkworm silk is mixed with PEO (polyethylene oxide) and electro spun. The resultant fibers have diameters of less than 800 nm. See Electrospinning Bombyx mori silk with poly(ethylene oxide) [Jin HJ, Fridrikh SV, Rutledge GC, Kaplan DL; "Electrospinning Bombyx mori silk with poly(ethylene oxide) "; Biomacromolecules; 2002 Nov-Dec; 3(6):1233-9 (www.ncbi.nlm.nih.gov/entrez/query.fcgi?CMD=Dispaly&DB=PubMed)].

Methods of isolating and purifying silk from the black fly, *Simulium vittatum* are described in US Patent No. 6642361.

An alternative fiber is that formed from a mixture of carbon nanotubes and spider silk or polymer. Carbon nano-tube reinforced silk is discussed by [Frank J, Ko, Ph.D., Material Sciences; "Carbon Nanotube Reinforced Silk"; Technology Marketing Abstract for Licensing Drexel University; Docket No. 03-0505D; pp 136-137; (http://www.research.drexel.edu/techcom/engine.asp?deva=Index)].

Spider silk fiber or the proteins from which such silk fiber is produced, are also described in US Patent Nos. 6268169, 6620917, 5994099, 5989894, 5756677, 5728810,5245012.

See also, A Spider's Yam [David Bradley; "A Spider's Yarn"; Instruments & Applications, American Chemical Society, 2001; (http://pubs.acs.org/subscribe/journals/tcaw/10/i03/html/03inst.htmal)].

The above lists are intended for illustrative purposes only, and not as a limitation on the scope of the present invention. The silk fiber suitable for use herein, can be obtained from a variety of sources.

Fiber strength may be characterized by, for one thing, tenacity (tensile-strength-to-weight-ratio) as measured in grams/denier (g/d). Fibers of any tenacity are within the scope of the present invention. Suitably, the fiber material employed herein exhibits a combination of high strength and good flexibility. Tenacities may range from about 5 g/d to 200 g/d and higher. Some fibers have tenacities in the range of about 5 g/d to about 40 g/d and more typically about 10 g/d to about 35 g/d. However, while some fibers may exhibit tenacities of about 5g/d to about 10g/d, others, such as dragline silk fibers, have extremely high tenacities of greater than about 40g/d up to about 200g/d or higher.

Silk fibers of both the synthetic and the natural type, can exhibit tenacities of about 2 times to about 6 times or more that of conventional man-made fibers. For example, Kevlar® fibers may be around 26 g/d and Spectra® fibers may be about 35 g/d.

Elongation to break of spider silk may be about 10% to about 50%, more suitably 10% to about 45%, with some having elongations of about 15% to about 20%, and other having elongations of about 30-35%. Some fibers have elongations of about 15% to about 20%, while some have elongations of about 30% to about 35%.

In some embodiments, the fibers employed have diameters of about less than 12 microns, suitably less than 11 microns, more suitably less than 10 microns, and most suitably in the range of about 2 microns to about 10 microns, while maintaining high strength and flexibility, although this range shall not be construed as limiting the diameter as such. One grade of Biosteel® spider silk is available in a thread size of about 7 microns in diameter.

In the embodiment shown in FIG. 3, balloon 10 is further shown with a matrix coating 16 applied over the fiber 14. The matrix coating may comprise any suitable material depending on the properties desired. The coating may be applied to the inner and/or outer surface of the web. For example, in those embodiments wherein an eliminatable shape-form is employed, a matrix material may be applied to both the inner and outer surface to encapsulate the fiber web. As is discussed in more detail below, shape-forms which can be eliminated after use through fluidizable or by non-fluidizable means may be employed in making the balloons described herein. Fluidizable shape-forms may be formed from compositions which may be meltable or dissolvable. Non-fluidizable shape-forms may include springs or coils, or may be deflated or broken after use, for example. Such shape-forms are described in more detail belowe.

The coating may be employed for any of a variety of reasons such as filling in any spaces which may be left as a result of wrapping the balloon with the fiber/thread, preventing penetration of water/saline (moisture resistance), encapsulation of thread/fiber structure, to increase balloon integrity and abrasion/puncture resistance, to increase lubricity, and so on and so forth. Combinations of materials may be employed in the matrix coating for providing combinations of properties as well. This coating may also carry therapeutic agent(s) such as an anticoagulant. Depending on the properties desired, any suitable material may be selected for this step, or any combination of materials may be selected for this step. Compatibility of the matrix material with the fiber may also be a factor in the selection of the matrix material. Compatibility refers to the solubility of a solid in a liquid, or the ability of solids to exist in intimate contact with one another over a long period with little adverse affect of one on the other. Of course, there are different levels of compatibility. One of ordinary skill in the art understands what is meant by this term.

Suitable materials, which may be employed in the matrix coating, include both thermoplastic and thermosetting materials including moisture cures and those cured through the use of actinic radiation such as UV radiation, and including both elastomers and non-elastomers, as well as mixtures thereof.

Examples of suitable matrix materials include, but are not limited to, polyimides, polyamides, polyesters, polyurethanes, polyethers, polyolefins, polyurethanes, polyalkylene oxides, copolymers thereof, and mixtures thereof.

Block copolymers are suitable for use as the matrix material and include, but are not limited to copolyester elastomers such as polyester-block-esters, polyester-block-ethers, and so forth; synthetic rubbers such as isoprene rubber, polybutadiene rubber, block copolymers having styrene endblocks and midblocks of isoprene, butadiene, isobutylene, ethylene/propylene, ethylene/butylene and so forth; polyamide copolymers such as polyether-block-amides; and so on and so forth.

Fibrous materials, cut or chopped to a shorter length, and then admixed in a solvent or cosolvent blend prior to application, may also be employed for the matrix coating. The fibrous material may be the same as or different then that used to form the web.

In some embodiments, it has been found beneficial to employ a matrix material having the same or similar chemical composition as that of the fiber web. For example, the same recombinant spider silk protein which is used for making the fiber may be employed in the matrix material. The recombinant spider silk protein may be applied to the fiber web out of a suitable solvent or cosolvent blend such as polyethylene oxide (PEO). The purified and spun spider silk, once the solvent has evaporated, forms a resilient coating which is not readily resolvated. Therefore, the spider silk proteins themselves, prior to being spun, may be employed as the matrix material. Without being bound by theory, it is surmised that there is hydrogen bonding between the protein molecules.

Solubilization of spider silk is discussed in US Patent No. 5245012.

Polyurethanes are also desirably employed for the matrix material and may dissolve in any suitable solvent. For example, alcohols may be employed as the solvent.

Mixtures of any of the above materials may also be employed in the coating.

These lists are intended for illustrative purposes only, and are not intended to limit the scope of the present invention.

Any method of applying the matrix coating known in the art may be employed including, but not limited to, spraying, rolling, painting, dipping, and so forth. The matrix material is suitably coated onto fiber web out of a solvent or a cosolvent blend. The solvent or cosolvent blend may be selected based on the type of matrix material selected for use. One of ordinary skill in the art would understand the selection of solvents. The matrix coating may substantially fix the fiber web to the balloon.

FIG. 4 is a partial cross-sectional side view taken at section A in FIG. 3 showing the first layer 12, which defines the shape of balloon 10, fiber 14, which forms a web over first layer 12, and a matrix coating 16.

Prior to application of the fiber 14 a material, which increases the frictional forces or otherwise adhesively binds the first layer 12 and the fiber 14 may be applied between the first layer 12 and fiber 14. This composition is typically different than that of matrix coating 16, although in some circumstances, they may be the same composition.

In one embodiment, a coating of an adhesive composition is applied to the fiber prior to application of the fiber to the balloon. Alternatively, the adhesive composition may be applied to the first layer. The composition may include any material, which increases the friction between the first layer and the fiber including both thermoplastic and/or thermosetting materials as well as elastomers and non-elastomers.

Any suitable polymeric composition which increases the amount of friction between the first surface 12 and the fiber 14 may be employed herein. This composition may be hereinafter referred to as an adhesive composition. Such adhesive compositions are known to those of skill in the art and include both thermoplastic and thermosetting materials such as those cured with actinic radiation including UV radiation and electron beam, and moisture curable compositions. Both elastomers and non-elastomers may be employed. Suitable examples include curing and non-curing elastomeric and non-elastomeric polyurethanes, polyamides, block copolymer elastomers including those having styrene endblocks such as styrene-isobutylene-styrene, natural gum rosins, and so forth, as well as mixtures thereof. This composition may substantially fix the fiber web to the balloon member and may be used in combination with or alternatively to said matrix coating. However, in some embodiments, neither an adhesive composition nor a matrix coating may be employed.

The fiber may be conveyed onto the first layer using any method known in the art such as braiding, weaving, wrapping or winding, roving, knitting, and so forth. As used herein, the term "web" shall hereinafter include braiding, weaving, knitting, roving, and so forth, as well as mesh-like structures, net-like structures, and so forth. Thus, the present invention is not limited by how the fiber web is configured onto the balloon.

In one method, the balloon structure in an inflated state, may be employed as a mandrel around which the fiber is wrapped, wound, braided, woven, or otherwise applied to the balloon structure.

Using such a method, the balloon may be mounted in a horizontal position, for example, for wrapping with fiber. A spool or pirn of fiber may be placed on a bobbin or bobbin-like structure, running it through an eyelet, and from the eyelet onto the balloon. The means of conveying the fiber onto the balloon can be any transport system known in the art. It is desirable, but not necessary, that the transport system create evenly spaced fibers. One method is to employ a left and right hand ground lead screw similar to that utilized on fishing reels to form a evenly spaced lay in one direction and then reverse with the same pitch.

In one embodiment the fiber is wrapped at preset distance on the balloon that is approximately equal to the diameter of the fiber, such that fiber contacts itself as it is wrapped around the balloon substantially encapsulating the balloon structure. Suitably, the fiber employed herein has a relatively small diameter of less than about 20 microns, and desirably less than 10 microns. However, larger diameter fibers may be suitable for some applications, and such a range is for illustrative purposes only, and not intended to limit the scope of the present invention.

The fiber may be wrapped one or more times around the balloon. In one embodiment the fiber is wrapped two times around the balloon. For example, the balloon may be wrapped helically at an angle to the longitudinal axis beginning at the right and going from right to left and then from left to right, or from left to right and then from right to left. In this embodiment, the effect is to substantially encapsulate the balloon by the fiber web. Such a two-ply structure has been found to exhibit high strength and durability. One method is to inflate the balloon, attach the fiber at one end, and rotate the balloon.

Alternatively, the fibers may also be chopped or cut into smaller pieces and applied to the balloon. The pattern may substantially cover or define the balloon structure, or partial cover may be suitable for some applications as well, such as just over the body of the balloon. A coating of an adhesive material may first be applied to the balloon structure or to a balloon preform. The fibers may be applied by blowing them onto the balloon or balloon preform. Chopped fibers may be aspirated from a chamber into a high volume air stream directed at the adhesive-coated balloon. Alternatively, the balloon or balloon preform may be rolled over a layer of fibers to create a monolayer type of coating on the balloon or balloon preform. These techniques result in a more random, thin layer of fiber material. If the fibers are applied to the balloon preform, then balloon molding will take place after application of the fibers.

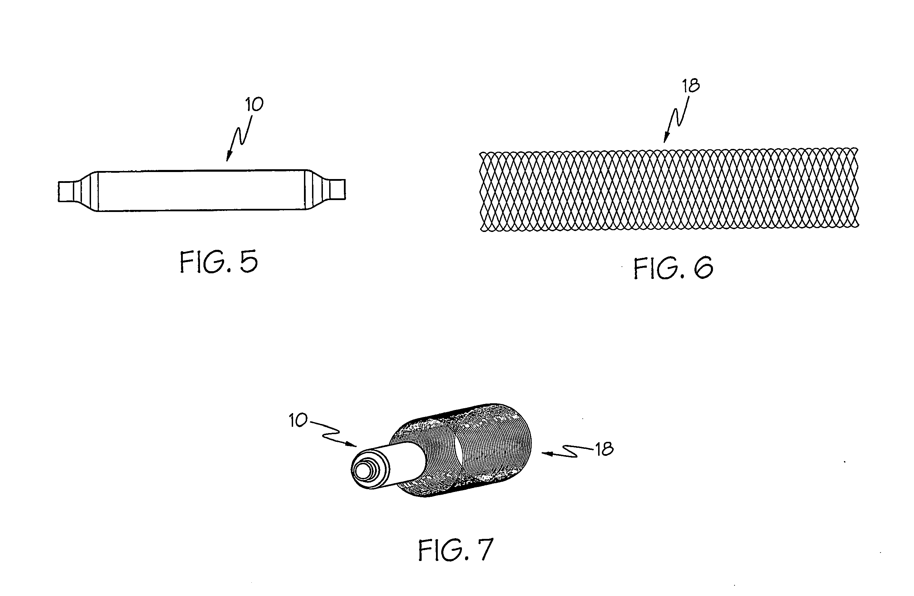

Alternatively, a pre-cut web in the form of a woven net or compressed mat of material may also be employed. In one embodiment, a web which is similar to a woven sock may be stretched over the balloon structure. FIGS. 5-7 illustrate such an embodiment. A molded balloon 10 shown in FIG. 5 and prepared using any conventional method as described above, is inserted into a preformed fiber web 18 shown in FIG. 6. FIG. 7 is an operable perspective view of balloon 10 being inserted into fiber web 18.

Alternatively, the balloon may be deflated, the sock placed over at least a portion of the balloon, and the balloon inflated into the sock.

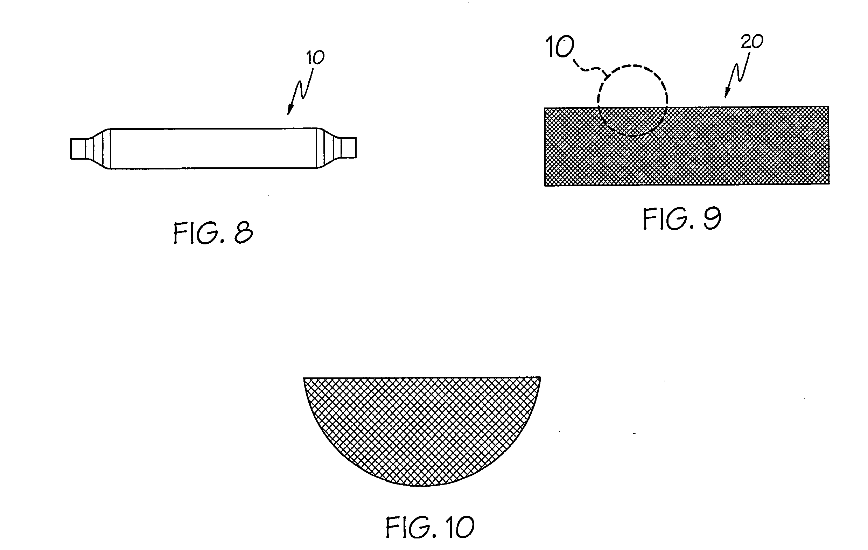

FIGS. 8-10 illustrate another embodiment wherein a fiber mat 20, shown in FIG. 9, is pre-formed and placed over a balloon 10 shown in FIG. 8. The mat may be formed over a mandrel to which is first applied an adhesive composition, such as a thermosetting urethane, for example. The adhesive composition may be applied using a fine spray mist, brushing, dipping, and so forth. Chopped fibers may then be applied over the adhesive composition also by spraying, or the mandrel may be rolled in a layer of chopped fibers. The chopped fibers may then be sprayed with another coating which is the same as the adhesive composition, or the fibers may be sprayed with a different composition, i.e. the matrix coating, as described above. Furthermore, the process may be repeated to build a desired mat thickness.

If a thermosetting composition is employed, it may be cured at an elevated temperature to firmly set the chopped fiber between the thermosetting composition. A thermosetting urethane may cure a temperature of about 68-70°C. A thermosetting composition, such as a urethane, may be advantageously employed because upon cure, the surface tack of the composition decreases.

The mat may also be formed in sheet form by applying a thin layer of chopped fibers to a film of adhesive composition. This process may also be repeated until a desired thickness has been reached. Curable compositions which lose tack upon curing may also be advantageously employed in such an embodiment. The mat may then be rolled into a tubular form, and the ends sealed prior to curing of the urethane composition, or through other conventional methods such as adhesively bonding or welding of the ends.

FIG. 10 is a more detailed view of the mat 20 taken at section A of FIG. 9.

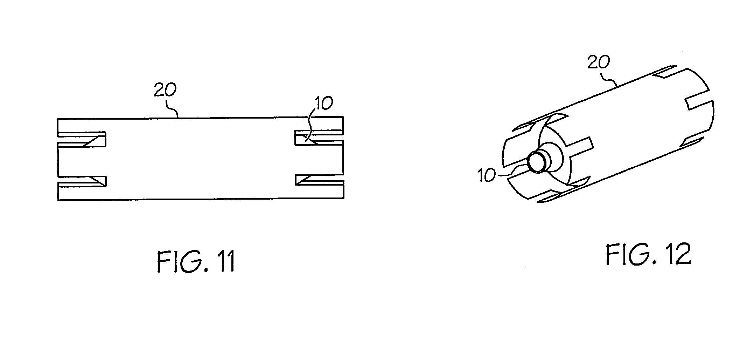

The balloon 10 may then be deflated and inserted into the finished mat 20 and the balloon then reinflated. FIGS. 11 and 12 are perspective views of the inflated balloon 10 shown inserted within mat 20. The ends of the mat may then be cut and the mat fashioned over the waist and cone portions of the balloon.

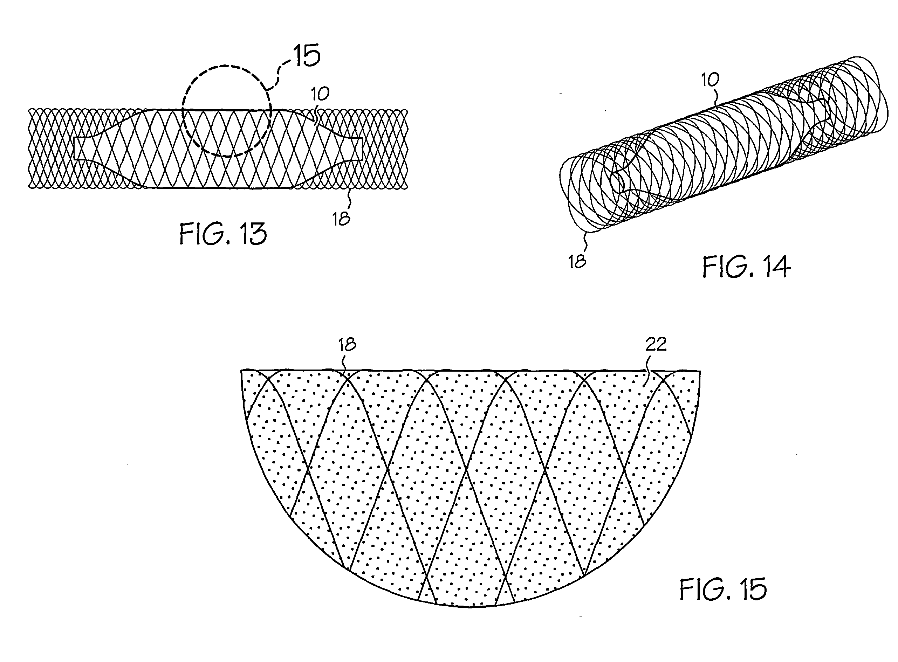

FIGS. 13-15 illustrate an embodiment in which a combination of chopped fibers and a preformed web are employed. In this embodiment, the chopped fibers may first be applied to a balloon structure. As described above, a friction-reducing composition or adhesive composition may be first applied to the balloon structure and the chopped fibers then applied by aspiration, by rolling the balloon in a layer of chopped fibers, and so forth. These methods are also discussed above. A fiber web may then be stretched over the inflated balloon structure shown in FIGS. 13-14. FIG. 15 shows a detailed view of the chopped fibers 22 and the fiber web 18 taken at section A of FIG. 13.

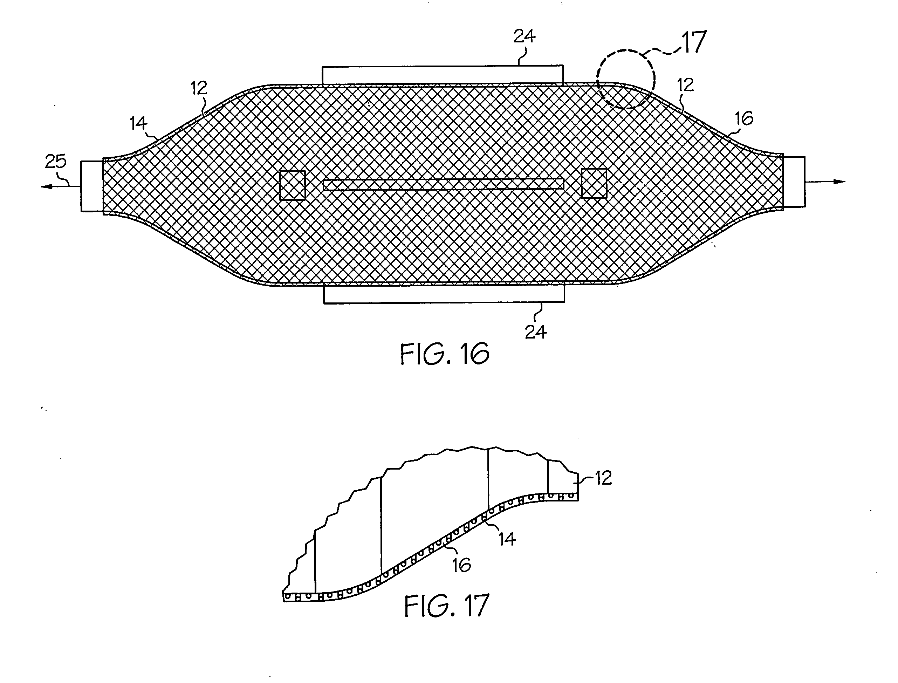

An alternative embodiment of the invention is shown in FIGS. 16-17. In this embodiment, balloon 10, is a cutting balloon shown with atherotomes 24 on the surface of balloon 10. Balloon 10 is defined by a first layer 12. First layer may be any conventional balloon material as discussed above. Typical balloon materials include polyalkylene terephthalates, polyethylene naphthalates, and polyamide copolymers such as poly (ether-block-amides). Over first layer 12, fiber 14 forms a web-like structure. In this particular embodiment, fiber 14 forms the web by wrapping the fiber 14 at an angle to the longitudinal axis 25 of balloon 10, i.e. in a helical manner about the balloon structure. The web may be wrapped once, twice and so forth. In this embodiment, the fiber web is shown wrapped helically about the balloon structure twice going from first from left to right and then from right to left or from right to left and then from left to right.

Over the web of fiber 14 is a matrix coating 16 as discussed above, resulting in fiber 14 being embedded between first layer 12 and matrix coating 16. In this embodiment, atherotomes or blades 24, are secured to the outer surface of balloon 10 using any method known in the art including adhesively bonding the atherotomes to the balloon surface. The blades 24 may be secured to balloon 10 prior to application of fiber 14. This can decrease the possibility of atherotomes 24 loosening from balloon surface 11. Again, fiber 14 may be wrapped as described above. Atherotomes 24 may be provided on balloon 10 either before or after application of the fiber web 14.

FIG. 17 is a detailed partial cross-sectional view taken at section C in FIG. 16 showing first layer 12, fiber 14 and matrix coating 16. As can be seen from the figure, the fiber is encapsulated.

In any of the above embodiments, the fiber web and/or chopped fibers may substantially cover or define the shape of the entire balloon structure, or partial cover may be suitable for some applications as well, such as just over the body of the balloon.

Furthermore, in any of the above embodiments, the fiber web and/or chopped fibers may be applied to a balloon preform. For example, a tube of the fiber web as shown in FIG. 6, may be placed over the balloon preform and the preform is then blow molded into a dilatation balloon using conventional balloon molding techniques as discussed above, essentially embedding the fiber web into the balloon. This step may substantially fix the fiber web to the balloon.

Other methods of substantially fixing the fiber web to the balloon may include, for example, heating at the interface between the fiber web and the balloon.

A shape-form which is eliminated after use by fluidization of the shape-form, or in some embodiments, by non-fluidization methods, may be employed in making the balloons according to the invention. The outer surface of the shape-form may determine the inner surface of the balloon or a balloon preform. Any of the embodiments of the fiber web described above may be applied to the shape-form. An adhesive composition may be first applied to the shape-form prior to application of the fiber web.

A coating of a matrix material, the same as or different than the adhesive composition, may then be applied over the fiber web.

The shape-form may then be eliminated. Means of eliminating the shape-form will depend on the type of material from which the shape-form is constructed.

Fluidization may be accomplished through dissolution, melting, and the like. Dissolution may be partial providing it is sufficient to allow the shape-form to be easily eliminated from the balloon.

Fluidizable shape-forms may include those which dissolve, or those which are meltable. Any polymeric material which is relatively low melting and/or which is readily dissolvable with a solvent may find utility herein. Waxes having low melting points such as paraffin waxes, ice, starch, sugar, waxes or other polymeric materials such as polyvinyl alcohol (PVOH), polyvinyl acetate (PVA), and so forth. Dissolution may be partial, providing that the material is reduced to a size which is small enough such as to be readily removable from the tubular member or balloon structure. This type of shape-form is also described in commonly assigned, copending application attorney docket number, S63.2-11491US01, Particularly suitable polymeric materials are those which are readily dissolvable with water.

The shape-form may be eliminated by non-fluidizable means. These types of shape-forms include, but are not limited to, coils or springs, such as those formed from copper, which when stretched, allow easy removal of the balloon. The spring can be pulled under tension and the resulting balloon structure or balloon preform allowed to slide free.

Other shape-forms eliminated by non-fluidizable means include those which may be deflated after use, or those which may be broken or shattered after use such as those formed from glass.

The balloons according to the invention may be employed with any suitable catheter assembly and include those used for angioplasty, those used in the biliary duct, urinary tract, cutting balloons, expandable members for medical delivery devices including stents, and so on and so forth. The balloons may also be employed with balloon-expandable medical device delivery assemblies such as those used for the delivery of stents.

By reinforcing medical balloons in this fashion, the likelihood of balloon rupture during use is decreased, without sacrificing balloon flexibility as can occur when balloon wall thicknesses are increased. Balloon rupture can be a potential problem with crossing of lesions during PTCA procedures, for example.

The present invention finds utility for other applications, however, such as restriction of balloon diameters for precise diameters, reinforcement of small medical device components such as small catheter tips, production of balloons having shaped surfaces such as those with ripples or tapered profiles, reinforcement of blade attachment in cutting balloons, reinforcement of thin wires used in probes for catheter assemblies, and so on and so forth.

In the case of diameter restriction of balloons, it may not be necessary to completely encapsulate the balloon, but rather to have a web with the fibers which have more spacing between each fiber.

For cutting balloons, the atherotomes or blades typically have a base or tab which is attached to the balloon body using standard securement methods, such as by adhesive attachment. The fiber may be wrapped over the base or tab, thereby minimizing the risk of detachment during use.

## Claims

1. An expandable medical balloon, the expandable medical balloon comprising:
a) a first layer having an inner surface and an outer surface defining the shape of the expandable balloon member; and
b) a web formed with silk fiber having an in inner surface and an outer surface, the web provided over at least a portion of said inner surface, said outer surface or both of said first layer.

2. The expandable medical balloon of claim 1 wherein the silk fiber is spun from recombinant silk proteins, from a silk producing member of the phylum Arthropoda or combination thereof.

3. The expandable medical balloon of claim 1 wherein said first layer is a polymer layer.

4. The expandable medical balloon of claim 3 wherein said polymer layer comprises a thermoplastic composition or a thermoset composition.

5. The expandable medical balloon of claim 1 further comprising an adhesive composition on said silk fiber, on said expandable medical balloon or both.

6. The expandable medical balloon of claim 5, said adhesive composition comprising at least one of a thermoplastic composition, a thermosetting composition, or mixture thereof.

7. The expandable medical balloon of claim 1 further comprising a matrix material on said outer surface of said web, said matrix material substantially encapsulating said web.

8. The expandable medical balloon of claim 7, said matrix material comprising at least one member selected from the group consisting of spider silk proteins, thermosetting materials, thermoplastic materials, lubricious materials, therapeutic agents and combination thereof.

9. The expandable medical balloon of claim 1, said first layer comprising at least one polymeric material selected from the group consisting of polyesters, polyethers, polyamides, polyurethanes, polyolefins, styrenic block copolymers, and mixtures thereof.

10. The expandable medical balloon of claim 9, said first layer comprising at least one of polyethylene terephthalate, polybutylene terephthalate, poly (ether-block-amide), poly (ether-block-ester), poly (ester-block-ester) or mixtures thereof.

11. The expandable medical balloon of claim 1, said expandable medical balloon further comprising atherotomes.

12. A process for forming an expandable medical balloon according to any of claims 1-11, the process comprising the steps of:
a) providing a shape-form, the shape-form defining the shape of the expandable medical balloon; and
b) providing a web of silk fibers over said shape-form, wherein said shape-form is fluidizable, the process further comprising the step of eliminating said shape-form after said step of providing a web of silk fibers over said shape-form, wherein said fluidizable shape-form is dissolvable.

13. The process of claim 12, wherein said fluidizable or non-fluidizable shape-form is a coil, spring, glass, starch, sugar, ice, polyvinyl alcohol, polyvinyl acetate or a combination thereof.

## Patentansprüche

1. Ausdehnbarer, medizinischer Ballon, wobei der ausdehnbare, medizinische Ballon umfasst:
a) eine erste Schicht, welche eine innere Oberfläche und eine äußere Oberfläche aufweist, welche die Form des ausdehnbaren Ballonbauteils definiert; und
b) ein Netz, welches aus Seidenfasern gebildet wird, welches eine innere Oberfläche und eine äußere Oberfläche aufweist, wobei das Netz, welches über zumindest einen Abschnitt der inneren Oberfläche, der äußeren Oberfläche oder beider der ersten Schichten bereitgestellt wird.

2. Ausdehnbarer, medizinischer Ballon nach Anspruch 1, wobei die Seidenfaser aus rekombinierenden Seidenproteinen, aus einem Seidenproduzierenden Bauteil der Phylum Arthoropoda oder einer Kombination davon gesponnen ist.

3. Ausdehnbarer, medizinischer Ballon nach Anspruch 1, wobei die erste Schicht eine Polymerschicht ist.

4. Ausdehnbarer, medizinischer Ballon nach Anspruch 3, wobei die Polymerschicht eine thermoplastische Komposition oder eine duroplastische Komposition umfasst.

5. Ausdehnbarer, medizinischer Ballon nach Anspruch 1, des Weiteren eine Haftkomposition auf der Seidenfaser, auf dem ausdehnbaren medizinischen Ballon oder beiden umfassend.

6. Ausdehnbarer, medizinischer Ballon nach Anspruch 5, wobei die Haftkomposition zumindest eine der thermoplastischen Komposition, der duroplastischen Komposition oder einer Mischung davon umfasst.

7. Ausdehnbarer, medizinischer Ballon nach Anspruch 1, des Weiteren ein Matrixmaterial auf der äußeren Oberfläche des Netzes umfassend, wobei das Matrixmaterial im Wesentlichen das Netz einkapselt.

8. Ausdehnbarer, medizinischer Ballon nach Anspruch 7, wobei das Matrixmaterial zumindest ein Element umfasst, welches aus der Gruppe gewählt wird, welche aus Spinnenseidenproteinen, Duroplastmaterialien, thermoplastischen Materialien, Schmiermaterialien, therapeutischen Wirkstoffen und Kombinationen davon besteht.

9. Ausdehnbarer, medizinischer Ballon nach Anspruch 1, wobei die erste Schicht zumindest ein polymerisches Material umfasst, welches aus der Gruppe gewählt wird, welche aus Polyestern, Polyethern, Polyamiden, Polyurethanen, Polyolefinen, styrenischen Blockpolymeren und Mischungen davon besteht.

10. Ausdehnbarer, medizinischer Ballon nach Anspruch 9, wobei die erste Schicht zumindest eine von Polyethylen-Therephthalat, Polybutlylen Therephthalat, Poly(ether-Block-Amiden), Poly(ether-Block-Ester), Poly(ester-Block-ester) oder Mischungen davon umfasst.

11. Ausdehnbarer, medizinischer Ballon nach Anspruch 1, wobei der ausdehnbare medizinische Ballon des Weiteren Atherotome umfasst.

12. Verfahren zum Bilden eines ausdehnbaren medizinischen Ballons nach einem der Ansprüche 1 bis 11, wobei das Verfahren die folgenden Schritte umfasst:
a) Bereitstellen einer Gestaltungsform, wobei die Gestaltungsform die Gestalt des medizinischen Ballons definiert; und
b) Bereitstellen eines Netzes aus Seidenfasern über die Gestaltungsform, wobei die Gestaltungsform verflüssigbar ist, das Verfahren des Weiteren den Schritt des Entfernens der Gestaltungsform nach dem Schritt des Bereitstellens eines Seidenfasernetzes über die Gestaltungsform umfasst, wobei die verflüssigbare Gestaltungsform lösbar ist.

13. Verfahren nach Anspruch 12, wobei die verflüssigbare oder nicht verflüssigbare Gestaltungsform eine Spule, eine Feder, Glas, Speisestärke, Zucker, Eis, Polyvinylalkohol, Polyvinylazetat oder eine Kombination davon ist.

## Revendications

1. Un ballonnet médical expansible, le ballonnet médical expansible comprenant :
a) une première couche ayant une surface intérieure et une surface extérieure définissant la conformation de l'organe de ballonnet expansible ; et
b) une trame formée de fibres de soie ayant une surface intérieure et une surface extérieure, la trame étant prévue sur au moins une partie de ladite surface intérieure, ladite surface extérieure ou l'une et l'autre de ladite première couche.

2. Le ballonnet médical expansible de la revendication 1, dans lequel la fibre de soie est filée à partir de protéines de soie recombinantes, à partir d'un organe producteur de soie du phylum Arthropoda ou d'une combinaison de ceux-ci.

3. Le ballonnet médical expansible de la revendication 1, dans lequel ladite première couche est une couche de polymère.

4. Le ballonnet médical expansible de la revendication 3, dans lequel ladite couche de polymère comprend une composition thermoplastique ou une composition thermodurcie.

5. Le ballonnet médical expansible de la revendication 1, comprenant en outre une composition adhésive sur ladite fibre de soie, sur ledit ballonnet médical expansible ou sur les deux.

6. Le ballonnet médical expansible de la revendication 5, où ladite composition adhésive comprend au moins l'une parmi une composition thermoplastique, une composition thermodurcissable ou un mélange de celles-ci.

7. Le ballonnet médical expansible de la revendication 1, comprenant en outre un matériau de matrice sur ladite surface extérieure de ladite trame, ledit matériau de matrice encapsulant substantiellement ladite trame.

8. Le ballonnet médical expansible de la revendication 7, où ledit matériau de matrice comprend au moins un élément choisi dans le groupe constitué par les protéines de soie d'araignée, les matériaux thermodurcissables, les matériaux thermoplastiques, les matériaux glissants, les agents thérapeutiques et les combinaisons de ceux-ci.

9. Le ballonnet médical expansible de la revendication 1, où ladite première couche comprend au moins un matériau polymère choisi dans le groupe formé par les polyesters, les polyéthers, les polyamides, les polyuréthannes, les polyoléfines, les copolymères à blocs styréniques, et des mélanges de ceux-ci.

10. Le ballonnet médical expansible de la revendication 9, où ladite première couche comprend au moins l'un parmi : téréphtalate de polyéthylène, téréphtalate de polybutylène, poly(éther-bloc-amide), poly(éther-bloc-ester), poly(ester-bloc-ester), ou des mélanges de ceux-ci.

11. Le ballonnet médical expansible de la revendication 1, où ledit ballonnet médical expansible comprend en outre des athérotomes.

12. Un procédé pour former un ballonnet médical expansible selon l'une des revendications 1 à 11, le procédé comprenant les étapes suivantes :
a) produire une forme de conformation, la forme de conformation définissant la conformation du ballonnet médical expansible ; et
b) produire une trame de fibres de soie sur ladite forme de conformation, ladite forme de conformation étant fluidisable, le procédé comprenant en outre l'étape d'élimination de ladite forme de conformation après ladite étape de production d'une trame de fibres de soie sur ladite forme de conformation, où ladite forme de conformation fluidisable peut être dissoute.

13. Le procédé de la revendication 12, dans lequel ladite forme de conformation fluidisable ou non fluidisable est une bobine, un ressort, du verre, de l'amidon, du sucre, de la glace, de l'alcool polyvinylique, de l'acétate polyvinylique ou une combinaison de ceux-ci.
